# EUROPEAN PATENT SPECIFICATION

(11) **EP 4 319 831 B1**
(45) Date of publication and mention of the grant of the patent: **03.06.2026**
(21) Application number: 22720667.9
(22) Date of filing: 05.04.2022
(51) Int. Cl.: B01D 61/00, A61M 1/16, B01D 65/10

(54) **DETERMINING A WATER PERMEABILITY STATUS OF A FORWARD OSMOSIS MEMBRANE USING TRANSMEMBRANE PRESSURE**
BESTIMMUNG EINES WASSERPERMEABILITÄTSSTATUS EINER VORWÄRTSOSMOSEMEMBRAN UNTER VERWENDUNG VON TRANSMEMBRANDRUCK
DÉTERMINATION D'UN ÉTAT DE PERMÉABILITÉ À L'EAU D'UNE MEMBRANE D'OSMOSE DIRECTE À L'AIDE D'UNE PRESSION TRANSMEMBRANAIRE

(30) Priority: 09.04.2021 US 202163172853 P; 12.08.2021 SE 2150995
(43) Date of publication of application: 14.02.2024
(73) Proprietor: Gambro Lundia AB, 226 43 Lund (SE)
(72) Inventor: VARTIA, Christian, 247 64 Veberöd (SE)
(74) Representative: Sweden SHS IP Office
(86) International application number: PCT/EP2022/058925
(87) International publication number: WO 2022/214449

(56) References cited:
- US-A- 4 202 764
- US-A- 5 476 592

## Description

### Technical field

The present disclosure relates to the field of dialysis and to water permeability of forward osmosis membranes, and in particular to determining a water permeability status of a forward osmosis membrane arranged in a dialysis solution generation apparatus.

### Background

Dialysis is commonly used for treating patients suffering from renal failure. Several types of dialysis treatments exist such as hemodialysis (HD), peritoneal dialysis (PD) and continuous renal replacement therapy (CRRT). Typically, a dialysis fluid is used in the treatment and is delivered ready-made in bags or is produced at the point of use by mixing concentrate and water.

Forward osmosis (FO) has emerged as an option for producing dialysis fluid as it has potential for reducing water consumption. A FO-membrane is typically designed to be more or less exclusively selective towards water molecules, which enables the FO-membrane to separate water from all other contaminants. However, undetected water permeability issues could alter the composition of the produced dialysis fluid by allowing transport of components other than water across the FO-membrane.

Thus, there is a need to detect such water permeability issues to not compromise the produced dialysis fluid.

US4202764 is considered as part of the prior art.

### Summary

A FO-membrane of the present disclosure is used to prepare dialysis fluid. The FO-membrane is in one embodiment more or less exclusively selective towards water molecules, which enables the FO-membrane to separate water from all other contaminants. An osmotic pressure difference between a feed fluid (for example water or effluent from the dialysis treatment) and a draw fluid (a dialysis concentrate) separated by the FO-membrane is used for extracting pure water from the feed fluid to the dialysis concentrate, thereby diluting the dialysis concentrate. The diluted dialysis concentrate is thereafter used for producing dialysis fluid. The systems and methods of the present disclosure detect water permeability issues, enabling alteration of the composition of the produced dialysis fluid by allowing transport of components other than water across the FO-membrane to be prevented.

It is accordingly an objective of the disclosure to provide an easy to perform and reliable method to determine a water permeability status of a forward osmosis membrane. It is a further objective to provide a method for determining a water permeability status of a forward osmosis membrane when the forward osmosis membrane is arranged for use in a dialysis fluid generation apparatus.

These objectives and others are at least partly achieved by the method, control arrangement and dialysis fluid generation apparatus according to the independent claims, and by the embodiments according to the dependent claims.

According to one aspect, which may be combined with any other aspect and embodiment thereof, the disclosure relates to a method for determining a water permeability status of a forward osmosis (FO)-membrane of a FO-device in a dialysis fluid generation apparatus. The FO-membrane separates a feed side from a draw side of the FO device. The FO-device comprises a feed inlet port and a feed outlet port in fluid communication with the feed side, and a draw inlet port and a draw outlet port in fluid communication with the draw side. The method comprises providing a flow of pure water at the feed side and providing a flow of pure water at the draw side. The method also comprises monitoring one or more pressures indicative of a transmembrane pressure (TMP) between the feed side and the draw side. The method further comprises stopping flow via one of the ports and controlling flow to or from the one of the feed side and the draw side that has a stopped flow based on the monitored one or more pressures, such that the TMP is maintained constant and at a non-zero magnitude. The method further comprises determining a water permeability status of the FO-membrane based on a property indicative of the controlled flow.

The method provides a straight-forward and reliable way to evaluate a water permeability status of a FO-membrane. By for example controlling a pump to maintain the TMP, the flow that the pump provides will reflect the flow of fluid transported between the sides and may reveal the water permeability status of the membrane. Alternatively, a resulting outflow from the other side will reflect the flow of fluid transported between the sides. The method is easy to implement as it makes use of already present mechanical features in the dialysis solution generation apparatus and can be performed automatically, without human intervention.

According to some embodiments, the method comprises monitoring the property indicative of the controlled flow. Here, the property may be continually observed, for example, continually measured.

According to some embodiments, controlling the flow comprises controlling flow with a pump. Here, a varied flow may be provided.

According to some embodiments, the property is a speed of the pump or power provided to the pump. The water permeability status may therefore be evaluated based on different properties.

According to some embodiments, the property is a flow rate of the controlled flow to the one of the feed side and the draw side that has a stopped flow; or the property is a flow rate of an outflow from the other one of the feed side and the draw side. The water permeability status may therefore be evaluated based on different properties.

According to some embodiments, the determining water permeability status of the FO-membrane comprises determining whether the property meets one or more criteria for a FO-membrane having an acceptable water permeability status. Here, the water permeability status may be determined based on what the property would be with a FO-membrane with an acceptable water permeability status.

According to some embodiments, the determining comprises determining that the FO-membrane has an acceptable water permeability status upon determining that the property is within or at a predetermined interval defining a FO-membrane having an acceptable water permeability status, or else determining that the FO-membrane has a water permeability error. The water permeability status may therefore be evaluated based on a comparison of the property with a predetermined interval for the property for a FO-membrane with an acceptable water permeability status.

According to some embodiments, the method comprises performing the method for both controlling flow such that the TMP is maintained positive and such that TMP is maintained negative, and wherein the determining further comprises determining a water permeability status of the FO-membrane based on a property indicative of the controlled flow for each case. Here, it is assured that a leakage that only causes a flow in one direction will be found.

According to a second aspect, which may be combined with any other aspect and embodiment thereof, the disclosure relates to a control arrangement for determining a water permeability status of a forward osmosis (FO)-membrane of a FO device in a dialysis fluid generation apparatus. The FO-membrane separates a feed side and a draw side of the FO device. The FO-device comprises a feed inlet port and a feed outlet port in fluid communication with the feed side, and a draw inlet port and a draw outlet port in fluid communication with the draw side. The control arrangement comprises a feed pump configured to provide a flow of pure water at the feed side, and a draw pump configured to provide a flow of pure water at the draw side. The control arrangement further comprises one or more valves configured to control an outflow from the feed side and the draw side, and one or more pressure sensors configured to sense a pressure indicative of a transmembrane pressure (TMP) between the feed side and the draw side. The control arrangement is configured to monitor one or more pressures indicative of the TMP. The control arrangement is further configured to stop flow via one of the ports and to control flow to the one of the feed side and the draw side that has a stopped flow, based on the one or more pressures, such that the TMP is maintained constant and at a non-zero magnitude. The control arrangement is further configured to evaluate a water permeability status of the FO-membrane based on a property indicative of the controlled flow.

According to some embodiments, the control arrangement is configured to perform a method according to any one of the embodiments described herein, in isolation or combination.

According to a third aspect, which may be combined with any other aspect and embodiment thereof, the disclosure relates to a solution generation apparatus for generating dialysis solution. The apparatus comprises a forward osmosis device comprising a FO-membrane that separates a feed side from a draw side of the FO device. The apparatus further comprises a control arrangement according to the second aspect, and optionally any embodiments thereof.

According to a fourth aspect, the disclosure relates to a computer program comprising instructions to cause the control arrangement according to the second aspect to execute the method according to the first aspect.

According to a fifth aspect, the disclosure relates to a computer-readable medium having stored thereon the computer program of the fourth aspect.

### Brief description of the drawings

Fig. 1 is a schematic illustration of a FO-device, according to some embodiments of the disclosure.
Fig. 2 illustrates an example dialysis fluid generation apparatus according to some embodiments of the disclosure.
Fig. 3 illustrates a simplified portion of the dialysis fluid generation apparatus in Fig. 2.
Figs. 4A-4D are a schematic illustration of the FO-device in Fig. 1, when flows via different ports are stopped and a flow to the side with a stopped port is controlled.
Fig. 5 illustrates a method for determining water permeability status of a FO-membrane of a FO-device according to some embodiments of the disclosure.

### Detailed description

In the following description, methods for determining a water permeability status of a FO-membrane will be described. The FO-membrane is used in a FO-device in a dialysis fluid generation apparatus for generating a dialysis solution that is thereafter used for producing a dialysis fluid. The dialysis fluid may be used for PD, HD, CRRT or any other dialysis treatment using dialysis fluid as a treatment fluid or replacement fluid (e.g., for diluting blood post-filtering).

In detail, the FO-membrane is used for extracting water from patient effluent, tap water or other water feed source into a dialysis concentrate to generate the dialysis solution. The FO-membrane may have a compromised permeability due to for example manufacturing errors, fouling or wear. The compromised permeability may be leakage (convective transport), or water permeability deterioration. Undetected integrity issues could alter the composition of produced dialysis fluid by allowing the transport of components other than water across the FO-membrane. For example, a leakage may allow the transport of microbials from the feed side (effluent or tap water) to the draw side (mixing side) and increase the risk for, e.g., peritonitis in the case of PD. Further, a leakage may allow transport of solutes (electrolytes, glucose, urea etc.) from the feed side (effluent or tap water) to the draw side (mixing side) and thereby alter the composition of the produced dialysis fluid. Water permeability deterioration may result in that less water molecules are transported through the intended water channels in the FO-membrane and that a dilution of a concentrate cannot be fully performed.

It has been found, as set forth in the present disclosure, that it is possible to detect leakage and/or water permeability deterioration after the FO-membrane is installed in the dialysis fluid generation apparatus, based on selectively stopping and controlling flow to and/or from the FO-device in a way that maintains a transmembrane pressure (TMP) over the FO-membrane constant. A property indicative of a resulting flow to maintain the TMP constant is investigated for determining a water permeability status of the FO-membrane. The fluids flowing at the feed side and draw side are pure water and hence have the same osmotic pressure and will not cause any osmotic pressure difference between the sides. Thereby, only water permeability is investigated. Tests for leakage from both feed side to draw side and draw side to feed side can be made to detect leaks that only allow convection in one direction. To determine water permeability status here means to determine if the FO-membrane has an acceptable water permeability status or a water permeability error, where the water permeability error is caused by leak and/or water permeability deterioration.

Generally, transport of water through the intended water channels is driven by the solute concentration difference, e.g., a concentration difference between feed side and draw side. Convective flow (caused by a leak) is driven by TMP, e.g., a pressure difference between the feed side and the draw side. However, water transport through the intended water channels is also driven by the TMP.

In some embodiments, the evaluation relies on using hardware already present in the apparatus and concentrates normally used for the production of dialysis fluid. For example, a pressure sensor is already present for sensing pressure at the feed side. The pure water used in the method is already present for producing dialysis solution.

The pure water typically has a quality as water for injection (WFI) or water for dialysis (WFD). WFI has a Total Organic Carbon (TOC) of maximum 500 ppg, a conductivity at 25°C of less than 1.3 µS/cm, and bacterial endotoxins at less than 0.25 EU/ml. WFD has a Colony Forming Unit (CFU) of less than 100 CFU/ml and an Endotoxin Unit of less than 0.25 EU/mL.

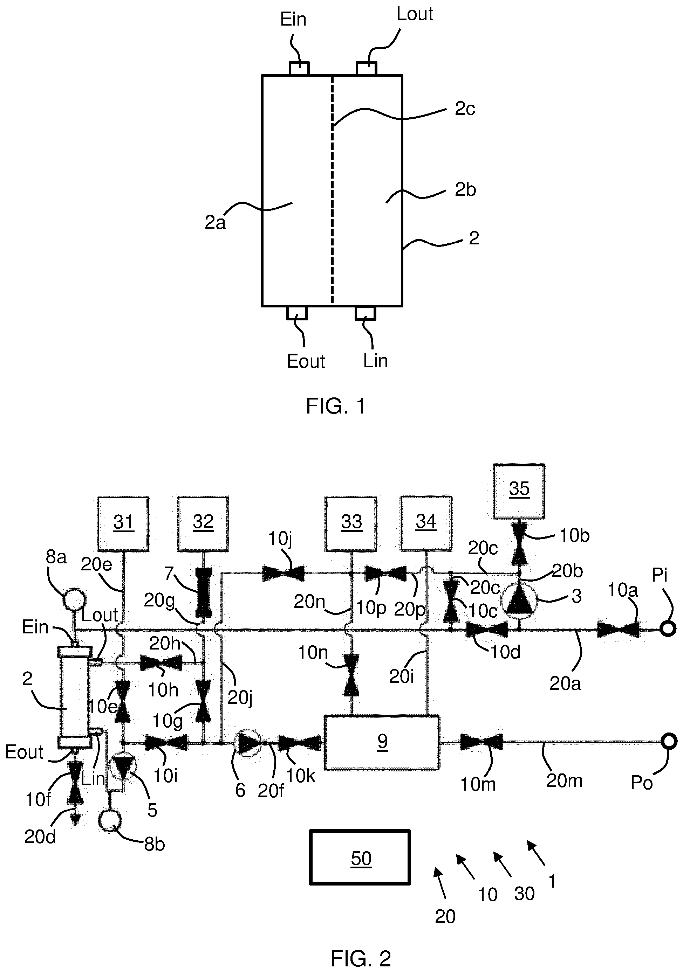

Embodiments of the disclosure will now be described with reference to Figs. 1 to 4. Fig. 1 is a schematic illustration of a FO-device 2 in isolation according to some embodiments. The FO-device 2 comprises a feed side 2a and a draw side 2b that are separated by a FO-membrane 2c. A side may also be referred to as a compartment or chamber. During use, the FO-membrane 2c separates a solution at the feed side 2a (referred to as a feed solution) and a solution at the draw side 2b (referred to as a draw solution). **In** the case of producing a dialysis solution, there is an osmotic pressure difference between the fluids, and the draw solution at the draw side "draws" pure water from the feed solution at the feed side. Hence, water is extracted from the feed solution at the feed side 2a to the draw solution at the draw side 2b. As a result, in the FO-process, the feed solution becomes dewatered, and the draw solution becomes diluted in the FO-device 2. The FO-membrane 2c is designed to be more or less exclusively selective towards permeating water molecules, which enables the FO-membrane 2c to separate water from all other contaminants. Hence, the FO-membrane 2c is a water permeable membrane. The FO-membrane 2c typically has a pore-size in the nanometer (nm) range, for example, from 0.5 to 5 nm or less depending on the solutes that are intended to be blocked. The FO-device 2 typically includes a cartridge that encloses the feed side 2a, draw side 2b and FO-membrane 2c. The geometry of the FO-membrane 2c may be a flat-sheet, or a tubular or hollow fiber. The feed side 2a has an inlet port *Eᵢₙ,* where a feed solution is passed into the feed side 2a, and an outlet port *Eₒᵤₜ,* wherefrom the feed solution is delivered from the feed side 2a. The draw side 2b has an inlet port *Lᵢₙ,* where a draw solution is passed into the draw side 2b, and an outlet port *Lₒᵤₜ,* wherefrom the draw solution is delivered from the draw side 2b. The fluids at these sides typically flow in counter-current flow but may alternatively flow in co-current flows. The flows may be continuous flows. Suitable FO-devices for FO-device 2 may be provided by, e.g., Aquaporin^{™}, AsahiKASEI^{™}, Berghof^{™}, CSM^{™}, FTSH₂O^{™}, Koch Membrane Systems^{™}, Porifera^{™}, Toyobo^{™} and Toray^{™}.

For producing a dialysis solution, the feed solution is for example effluent from a previous or current dialysis treatment or water. The draw solution is for example a dialysis concentrate, and the extracted water from the feed solution dilutes the dialysis concentrate into a dialysis solution, which may also be referred to as a "diluted dialysis concentrate", an "intermediate dialysis solution"," or simply "dialysis solution". The dialysis concentrate is for example a concentrate including at least one of, e.g., a plurality of, NaCl, KCl, CaCl2, MgCl2, HAc, glucose, lactate and bicarbonate. For example, the dialysis concentrate may comprise NaCl, CaCl2, MgCl2 and Na-lactate. For determining the water permeability status of the FO-membrane 2c, the feed solution and the draw solution are pure water.

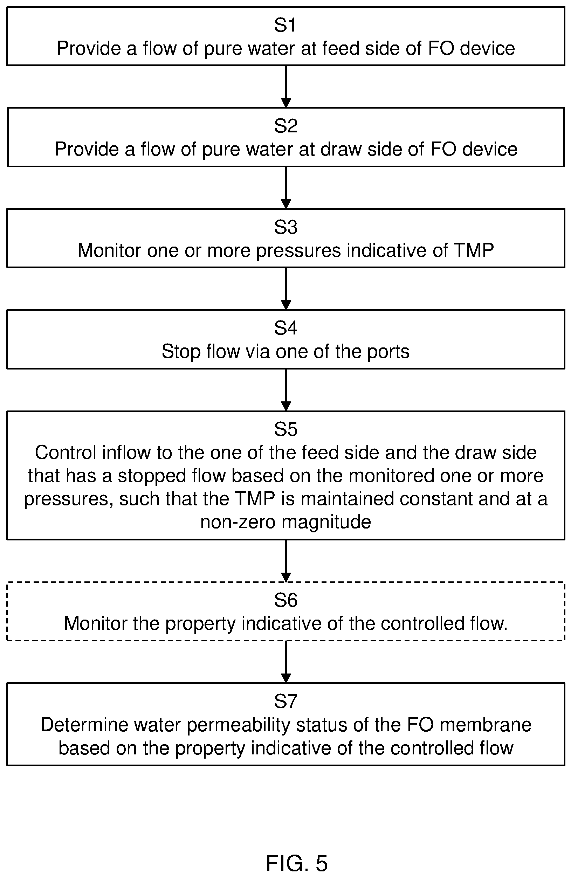

Fig. 2 illustrates a dialysis fluid generation apparatus 1 (hereinafter "apparatus 1") according to some embodiments of the disclosure. The apparatus 1 comprises a FO-device 2 as explained with reference to Fig. 1. The apparatus 1 also comprises a flow path 20 comprising a plurality of fluid lines 20a-20n, hereinafter referred to as "lines". The apparatus 1 further comprises a control arrangement 30. The control arrangement 30 comprises a feed pump 3, a draw pump 5 and a diluted concentrate pump 6. A pump is for example a volumetric pump (such as a piston pump) or a non-volumetric pump (for example a gear pump) with flow rate feedback from a flow sensor (not shown). The feed pump 3 is configured to pump effluent from a patient connected to an inlet connector *Pᵢ* to an effluent container 35. The feed pump 3 is configured to provide a flow of effluent from the effluent container 35 or inlet connector *Pᵢ* to the first side 2a and from the first side 2a to a drain (not shown). The draw pump 5 is configured to provide a flow of a dialysis concentrate from a dialysis concentrate container 31 to the second side 2b, and further pump the generated solution at the second side 2b to a diluted concentrate container 32. A pure water container 33 comprises pure water. A fluid container 34 comprises an osmotic agent or a buffer solution, for example, glucose solution or bicarbonate solution. The apparatus 1 also comprises a conductivity sensor 7 configured to sense a conductivity of a solution generated from the second side 2b. The conductivity sensor 7 is typically arranged to sense conductivity in the range of 0.1 to 40 mS/cm. The conductivity sensor may 7 also include a temperature sensor (not shown) to compensate sensed conductivity values. The apparatus 1 further comprises one or more pressure sensors configured to sense pressures indicative of a TMP of the FO-device 2. For example, the apparatus 1 comprises a first pressure sensor 8a arranged to sense a pressure at the feed side 2a. The apparatus 1 may also comprise a second pressure sensor 8b arranged to sense a pressure at the draw side 2b. The apparatus 1 may however include additional pressure sensors for sensing pressures indicative of TMP, or other pressure sensors used for producing dialysis fluid. The diluted concentrate pump 6 is configured to provide a flow of fluid in a main line 20f. The control arrangement 30 also comprises a valve arrangement 10 comprising a plurality of valves 10a-10p. Generally, a valve connected to a line may be configured as open, wherein a fluid flow in the line is allowed, and closed, wherein a fluid flow in the line is stopped. A valve may for example be an on/off valve, wherein the on state defines a state when fluid flow in the line is allowed, and the off state is a state wherein the fluid flow in the line is stopped. The control arrangement 30 further comprises a control unit 50 comprising at least one memory and at least one processor. The control arrangement 30, by means of the control unit 50, is configured to control the pumps, the valves of the valve arrangement 10 and the functionality of a mixing unit 9, to perform a plurality of different processes, such as to dilute a dialysis concentrate into dialysis solution, provide dialysis fluid, perform a cleaning process or a priming process. The control arrangement 30 is also configured to receive measurements of conductivity from the conductivity sensor 7. The control arrangement 30 is further configured to receive measurements of pressure from the one or more pressure sensors, including the first and second pressure sensors 8a, 8b. The control arrangement 30 is further configured to determine a TMP of the FO-device 2, based on the measurements of pressure from the one or more pressure sensors. For example, the control arrangement 30 is configured to determine the TMP as a hydrostatic pressure P_{feed} at the feed side 2a minus the hydrostatic pressure P_{draw} at the draw side 2b (TMP = ΔP = P_{feed} - P_{draw}). The control arrangement 30 is further configured to determine the water permeability status of the FO-membrane 2c in the apparatus 1, defined by a method illustrated in Fig. 5. For that purpose, the at least one memory comprises computer instructions for determining water permeability status of the FO-membrane 2c. When the instructions are executed by the at least one processor, the control arrangement 30 performs the method for determining a water permeability status of the FO-membrane 2c, which is explained below. The method may be performed by the control arrangement 30 and stored as a computer program including computer instructions on the at least one memory.

However, first the apparatus 1 in Fig. 2 will be described in more detail. In Fig. 2, a first effluent inlet line 20a is arranged between the inlet connector *Pᵢ* and a feed inlet port *Eᵢₙ* of the first side 2a, to connect the inlet connector *Pᵢ* and the feed inlet port *Eᵢₙ.* The inlet connector *Pᵢ* is for example connectable to a catheter of a PD patient, or to an effluent line of a HD or CRRT apparatus. A first effluent inlet valve 10a is connected to the first effluent inlet line 20a. A second effluent inlet line 20b is arranged between the first effluent inlet line 20a and the effluent container 35, to connect the first effluent inlet line 20a and the effluent container 35. The feed pump 3 is arranged to provide a flow of effluent in the second effluent inlet line 20b. A second effluent inlet valve 10b is connected to the second effluent inlet line 20b. A third effluent inlet line 20c is arranged between the first effluent inlet line 20a and the second effluent inlet line 20b, to connect the first effluent inlet line 20a and the second effluent inlet line 20b. A third effluent inlet valve 10c is connected to the third effluent inlet line 20c. A fourth effluent inlet valve 10d is connected to the first effluent inlet line 20a between the connection of the second effluent inlet line 20b and the third effluent inlet line 20c to the first effluent inlet line 20a. The effluent may be collected in the effluent container 35 by pumping effluent to the container 35 with the feed pump 3 from the inlet connector *Pᵢ*, opening first effluent inlet valve 10a and second effluent inlet valve 10b, and closing third effluent inlet valve 10c and fourth effluent inlet valve 10d. Effluent can thereafter be pumped with feed pump 3 from effluent container 35 to feed side 2a by opening second effluent inlet valve 10b and fourth effluent inlet valve 10d, and closing first effluent inlet valve 10a, third effluent inlet valve 10c and second water valve 10p. Alternatively, effluent may be pumped directly to first side 2a by pumping effluent with feed pump 3 from inlet connector *Pᵢ* through first effluent inlet line 20a, second effluent inlet line 20b and third effluent inlet line 20c, and opening first effluent inlet valve 10a and third effluent inlet valve 10c, and closing second effluent inlet valve 10b, fourth effluent inlet valve 10d and second water valve 10p.

An effluent outlet line 20d is arranged between the feed outlet port *Eₒᵤₜ* of the first side 2a and a drain (not shown) and connects the feed outlet port *Eₒᵤₜ* of the first side 2a to drain. A drain valve 10f is connected to the effluent outlet line 20d. Hence, the FO-device 2 comprises a feed inlet port *Eᵢₙ* and a feed outlet port *Eₒᵤₜ* in fluid communication with the feed side 2a.

Further, a dialysis concentrate line 20e is arranged between the dialysis concentrate container 31 and the draw inlet port *Lᵢₙ* of the draw side 2b to connect the electrolyte solution container 31 and the draw inlet port *Lᵢₙ* of the draw side 2b. The draw pump 5 is arranged to provide a flow in the dialysis concentrate line 20e. A main line 20f is arranged between the dialysis concentrate line 20e and a mixing unit 9 and connects the dialysis concentrate line 20e and the mixing unit 9. The mixing unit 9 includes fluid mixing functionality such as a main pump controlling a resulting flow rate in line 20m located downstream mixing unit 9, a fluid pump providing a flow of osmotic agent or buffer from the fluid container 34, a conductivity sensor, a heater and a mixing chamber (these features not explicitly shown). The main line 20f is connected to the dialysis concentrate line 20e between the dialysis concentrate container 31 and the draw pump 5. A diluted dialysis concentrate line 20g is arranged between a diluted dialysis concentrate container 32 and the main line 20f, to connect the diluted dialysis concentrate container 32 and the main line 20f. The conductivity sensor 7 is connected to the diluted dialysis concentrate line 20g to sense a conductivity of the fluid in the diluted dialysis concentrate line 20g, and hence the conductivity of a fluid in the dialysis concentrate container 32. A diluted dialysis concentrate valve 10g is connected to the diluted dialysis concentrate line 20g. A first connecting line 20h is arranged between the draw outlet port *Lₒᵤₜ* of the draw side 2b and the diluted dialysis concentrate line 20g, to connect the draw outlet port *Lₒᵤₜ* of the draw side 2b and the diluted dialysis concentrate line 20g. A first connecting valve 10h is connected to the first connecting line 20h. Hence, the FO-device 2 comprises a draw inlet port *Lᵢₙ* and a draw outlet port *Lₒᵤₜ* in fluid communication with the draw side 2b. A first main valve 10i is connected to the main line 20f, between a connecting point of the main line 20f and the dialysis concentrate line 20e, and a connecting point of the diluted dialysis concentrate line 20g and the main line 20f. A concentrate valve 10e is connected to the dialysis concentrate line 20e between the dialysis concentrate container 31 and the draw pump 5. Dialysis concentrate may be pumped from the dialysis concentrate container 31 to the diluted dialysis concentrate container 32 via the draw side 2b, by pumping with draw pump 5, opening concentrate valve 10e and first connecting valve 10h, closing diluted dialysis concentrate valve 10g and first main valve 10i. Simultaneously, effluent may be pumped at the feed side 2a. Pure water is extracted from the effluent at the feed side 2a to the dialysis concentrate at the draw side 2b, by osmotic pressure. Thus, the dialysis concentrate becomes diluted to form an intermediate dialysis solution and is collected in diluted dialysis concentrate container 32. This procedure may be referred to as a FO-session. Hence, the FO-device 2 is configured to be used in a FO-session for diluting a dialysis concentrate in a process of producing a dialysis solution.

A fluid line 20i is arranged between the fluid container 34 and the mixing unit 9, to connect the fluid container 34 and the mixing unit 9. A second main valve 10k is connected to the main line 20f, between the diluted concentrate pump 6 and the mixing unit 9. A first water line 20n is arranged between the pure water container 33 (containing pure water) and the mixing unit 9, to connect the pure water container 33 and the mixing unit 9. A first water valve 10n is connected to the first water line 20n. An outlet line 20m is arranged between the mixing unit 9 and an outlet connector *P*ₒ, to connect the mixing unit 9 and the outlet connector *Pₒ.* The outlet connector *Pₒ* may for example be connected to a catheter of a PD patient, or to a dialysis fluid line of a HD or CRRT apparatus. An outlet valve 10m is arranged to the outlet line 20m.

For mixing a dialysis fluid, the diluted dialysis concentrate solution in diluted dialysis concentrate container 32 is pumped to mixing unit 9 by pumping with diluted concentrate pump 6, opening diluted dialysis concentrate valve 10g, second main valve 10k and outlet valve 10m. At the same time, osmotic agent or buffer is passed to the mixing unit 9 from the fluid container 34 via fluid line 20i by pumping with the fluid pump (not shown). Pure water flows to the mixing unit 9 via first water line 20n. The main pump (not shown) provides a desired flow rate of resulting dialysis fluid in the line 20m downstream mixing unit 9. A conductivity sensor (not shown) of the mixing unit 9 measures the conductivity of the resulting dialysis fluid from the mixing unit 9. The diluted concentrate pump 6 and the fluid pump are controlled to certain speeds to achieve a desired predetermined concentration of the resulting dialysis fluid, based on the conductivity of the produced fluid, the conductivity of the diluted dialysis concentrate solution and flow rate of the produced fluid. **In** the mixing unit 9, the diluted dialysis concentrate solution, the osmotic agent/buffer and the pure water are mixed in a mixing chamber to form a dialysis fluid, and are optionally heated. Thereafter, the dialysis fluid is delivered at the outlet connector *Pₒ* via outlet line 20m to a desired destination (e.g., a storage container or a dialysis machine).

A second water line 20p is arranged between first water line 20n and third effluent line 20c. The second water line 20p thus connects the first water line 20n and third effluent line 20c. The second water line 20p connects to the first water line 20n between the pure water container 33 and the first water valve 10n. The second water line 20p further connects to the third effluent line 20c between the third effluent inlet valve 10c and the second effluent line 20b. A second water valve 10p is connected to the second water line 20p. A third water line 20j is arranged between the first water line 20n and the main fluid line 20f. The third water line 20j thus connects the first water line 20n and the main fluid line 20f. A third water valve 10j is connected to the third water line 20j. The third water line 20j connects to the first water line 20n between the pure water container 33 and the first water valve 10n. The third water line 20j further connects to the main line 20f between the diluted concentrate pump 6 and the first main valve 10i. Pure water may thus be passed from the pure water container 33 to the feed side 2a, and from the feed side 2a to drain (not shown), via the second fluid line 20p, the third effluent line 20c, the second effluent line 20b and the first effluent line 20a by pumping pure water with the feed pump 3, opening second water valve 10p and fourth effluent inlet valve 10d, and closing second effluent inlet valve 10b, first effluent inlet valve 10a, and third effluent inlet valve 10c. Pure water may simultaneously be passed from pure water container 33 to the draw side 2b, and from draw side 2b to the diluted dialysis concentrate container 32, via the third water line 20j, main fluid line 20f, dialysis concentrate line 20e, connecting line 20h and diluted dialysis concentrate line 20g. The third water valve 10j, first main valve 10i, first connecting valve 10h are then open, and concentrate valve 10e and diluted dialysis concentrate valve 10g are closed. The pure water may thereafter be pumped to drain via a drain connection (not shown).

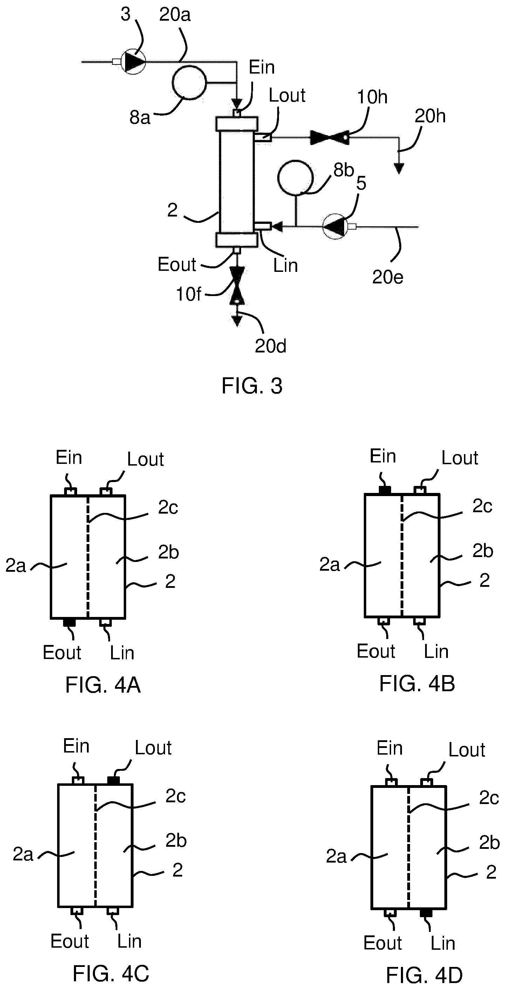

Fig. 3 illustrates a simplified part of the dialysis fluid generation apparatus in Fig. 2, with some relevant parts for performing the following method (except for the control unit 50).

A method for determining a water permeability status of a FO-membrane will now be explained with reference to the schematic illustrations of the FO-devices in Figs. 4A to 4D, and to the flow chart in Fig. 5. Figs. 4A to 4D illustrate the FO-device in Fig. 1 when flow via different ports is stopped and a flow is controlled to the side with a stopped flow via a port. The port with a stopped flow is indicated with the port filled in black. The schematics illustrate which flow is stopped in different embodiments of the method that will be explained in the following. The method is for example implemented by the control unit 50 in Fig. 2. The FO-membrane is for example the FO-membrane 2c of the FO-device 2 in the apparatus 1 in Fig. 2 or 3. However, the method may be used in other apparatuses comprising a FO-membrane for determining the water permeability status thereof. The method comprises providing S1 a flow of pure water at the feed side 2a. In other words, the method comprises passing pure water at the feed side 2a. Hence, pure water provided at the feed inlet port *Eᵢₙ* flows from the feed inlet port *Eᵢₙ,* through the first side 2a to the outlet port *Eₒᵤₜ,* where the solution leaves the FO-device 2. For the example in Fig. 2, providing S1 includes pumping pure water from the pure water container 33 and to the inlet port *Eᵢₙ* of the first side 2a, using the feed pump 3 and opening/closing appropriate valves. The operating point is typically well defined, hence, providing S1 includes providing the pure water at a constant relatively high flow rate to the feed side 2a. For PD, the flow rate provided with the feed pump 3 is for example between 50 to 200 ml/min. For HD, the flow rate provided with the feed pump 3 is for example between 200 to 600 ml/min. The flow rate is controlled directly with the feed pump 3 or measured with a flow sensor (not shown) and used as feedback for flow rate control with the feed pump 3. The hydrostatic pressure P_{feed} at the feed side 2a may simple be a result of providing a certain flow rate as detailed above. Alternatively, providing S1 may include providing the pure water with a hydrostatic pressure at the feed side 2a that is at or close to atmospheric pressure, or different from (higher or lower) than the hydrostatic pressure at the draw side 2b. The pressure is for example controlled using the feed pump 3 and/or the drain valve 10f. The pressure at the feed side may be measured with the first pressure sensor 8a and used as feedback for pressure control with the feed pump 3 and/or drain valve 10f. The method also comprises providing S2 a flow of pure water at the draw side 2b. Hence, the draw solution and the feed solution have the same osmotic pressure. As the flows are of pure water, the flows have a very low osmotic pressure, well below 1 bar (1 bar = 100.000 Pascal) (14.5 psig). Providing S2 of a flow of pure water at the draw side 2b is performed while providing S1 a flow of pure water at the feed side 2a. In other words, the method comprises passing pure water at the second side 2b. Hence, the pure water provided at the draw inlet port *Lᵢₙ,* flows from the draw inlet port *Lᵢₙ,* through the second side 2b to the draw outlet port *Lₒᵤₜ,* where the pure water leaves the FO-device 2. The operating point of the apparatus 1 is typically well defined while providing S2 a flow of pure water. For example, the operating point includes providing S2 a flow of pure water with a constant flow rate of pure water provided to the draw side 2b. The flow rate provided with the draw pump 5 is typically the same flow rate used for the feed solution. In the example in Fig. 2, providing S2 comprises pumping pure water from pure water container 33 to the draw inlet port *Lᵢₙ* of the second side 2b, using the draw pump 5. A hydrostatic pressure P_{draw} at the draw side 2b is typically at or close to atmospheric pressure, for example, 1013 hPa (about 1 bar, 14.5 psig). This is because the draw side 2b and the diluted concentrate container 32 are fluidly connected, which means that they are also at about the same pressure, except for a potential hydrostatic difference. Providing S1, S2 flows of pure water to the draw side and at the feed side are performed at flow rates that ensure that all possible osmotic pressure generating solutes are flushed out to obtain a good starting point for the following method steps, in which the osmotic pressure at both sides (feed side and draw side) are equal. Hence, the osmotic pressure difference between the sides is zero. Thus, there is no need to apply a certain hydrostatic pressure at any of the sides at this stage.

After flows at both the feed side 2a and the draw side 2b are provided, method steps may be repeatedly performed to determine a water permeability status from either feed side 2a to draw side 2b, or from draw side 2b to feed side 2a. For example, a leakage in either direction may be detected. Generally, by stopping flow through a port from one side, either the feed side 2a or the draw side 2b, and controlling flow to or from the same one side that has a stopped flow, via the other port to maintain a constant TMP, the controlled inflow or outflow reflects the flow through the FO-membrane 2c. Hence, in all embodiments, the method may comprise monitoring S3 one or more pressures indicative of a transmembrane pressure (TMP) between the feed side 2a and the draw side 2b. The TMP may be determined as ΔP = P_{feed} - P_{draw}. As the hydrostatic pressure at the draw side 2b may remain at around atmospheric pressure if the draw side is connected to the diluted concentrate container 32 or other container or fluid line, which are in turn connected to atmospheric pressure. Here, the TMP may be derived from the hydrostatic pressure P_{feed} at the feed side 2a only. Hence, monitoring S3 may include measuring the pressure at the feed side 2a, using, e.g., the first pressure sensor 8a, and using the measured pressure at the feed side 2a as an estimation of the TMP. Alternatively, the monitoring S3 may include measuring the pressure at the feed side 2a (using first pressure sensor 8a) and measuring the pressure at the draw side 2b (using second pressure sensor 8b) and determining the TMP as P_{feed} - P_{draw}.

The testing of water permeability by stopping and controlling flow to/from the feed side 2a will now be explained, with reference to Figs. 4A, 4B and 5. To perform such testing, in a first embodiment, the method comprises stopping S4 flow via the feed outlet port *Eₒᵤₜ.* The stopping of the flow via the feed outlet port *Eₒᵤₜ* is illustrated in Fig. 4A with a filled outlet port *Eₒᵤₜ.* **In** the examples in Figs. 2 and 3, the flow via the feed outlet port *Eₒᵤₜ* can be stopped by closing the drain valve 10f. Through the other ports, in one embodiment, flow is allowed, as illustrated in Fig. 4A by being not filled. Hence, the flows via the draw outlet port *Lₒᵤₜ* and the draw inlet port *Lᵢₙ* remain unstopped, and typically remain at the same flow rate and pressure as during the step S2. **In** an alternative embodiment, the flow through the draw inlet port *Lᵢₙ* is also stopped, e.g., by stopping pumping with draw pump 5. The method further comprises controlling S5 flow via, either inflow to or outflow from, the feed inlet port *Eᵢₙ* based on the monitored one or more pressures, such that the TMP is maintained constant and at a non-zero magnitude. Controlling S5 is for example performed using the feed pump 3. To maintain the TMP constant means controlling the TMP such that a predetermined TMP value is achieved, by means of controlling the inflow or outflow to the feed side 2a. Having a non-zero TMP means that the hydrostatic pressures at the sides 2a, 2b are different, and that there is a TMP that drives the water transport. The TMP may be either positive or negative. Depending on the positive or negative sign of the predetermined TMP that the TMP is to be controlled to, either the inflow or the outflow is controlled to maintain the TMP constant at the predetermined TMP. As it is only the TMP that drives the water transport through the FO-membrane 2c, by changing the positive or negative sign of the TMP, the water transport can be made to progress in different directions. Hence, if TMP is positive, then P_{feed} is greater than P_{draw} and the TMP is driving the water transport from the feed side 2a to the draw side 2b. The predetermined TMP is then for example from 2 to 5 bar (29 to 72.5 psig), for example, 4 bar (58 psig). The only way water at the feed side that can leave the feed side 2a is via the FO-membrane 2c to the draw side 2b, via a leakage and/or via water permeation, through the intended water channels in the FO-membrane 2c. The pressure at the feed side 2a will then decrease, wherein water is pumped into the feed side 2a to maintain the TMP constant at the predetermined TMP. Hence, in some embodiments, the method includes controlling S5 inflow to the feed inlet port *Eᵢₙ* based on the monitored one or more pressures, such that the TMP is maintained at a positive predetermined TMP. Alternatively, the TMP can be negative, which means that P_{draw} is greater than P_{feed}, and the TMP is driving the water transport from the draw side 2b to the feed side 2a. The predetermined TMP is then for example from -0.5 to -2 bar (-7.3 to -29 psig), for example, -1 bar (-14.5 psig). Water at the draw side 2a can now leave the draw side 2b via the FO-membrane 2c to the feed side 2b, via a leakage and/or via water permeation through the intended water channels in the FO-membrane 2c. However, the pressure at the feed side 2a will then increase, wherein water is pumped out from the feed side 2a to maintain the TMP constant. Hence, in some embodiments, the method includes controlling S5 outflow from the feed inlet port *Eᵢₙ* based on the monitored one or more pressures, such that the TMP is maintained at a negative predetermined TMP. The TMP value used for the control is a predetermined TMP, determined, e.g., based on experimentation and/or calculations. The predetermined TMP may also be determined by the manufacturer of the FO-membrane as a stated maximum pressure difference between the feed side and the draw side for the FO-membrane. P_{draw} typically remains at around atmospheric pressure. By controlling the inflow or outflow to the feed side 2a to maintain the TMP constant, the flow to or from the feed side 2a reflects an eventual flow through a leakage and/or membrane water permeation of the FO-membrane 2c.

Stopping S4 flow via the feed outlet port *Eₒᵤₜ* and controlling S5 inflow to or outflow from the feed inlet port *Eᵢₙ* are performed simultaneously in one embodiment. After a short time period of stabilization, the inflow to or outflow from the feed inlet port *Eᵢₙ* reveals if there is a leakage and/or altered membrane permeability. The inflow or outflow may be determined based on a property indicative of the controlled inflow or outflow, respectively. **In** some embodiments, the property is a speed of the feed pump 3 or power provided to the feed pump 3. **In** some embodiments, the property is a flow rate of the controlled flow provided by the feed pump 2a. Such properties may be readily available as control parameters or other parameters in the control arrangement 50 to be used for an evaluation. The flow rate of the controlled flow may alternatively be measured with a flow sensor (not shown). A water transport from the feed side 2a to the draw side 2b will be detected as an increased flow through the draw outlet port *Lₒᵤₜ* compared to the flow through the draw inlet port *Lᵢₙ* (provided with the draw pump 5). The difference between the flow rate of the fluid from the draw outlet port *Lₒᵤₜ* and the flow rate of the fluid to the draw inlet port *Lᵢₙ* indicates, e.g., be equal to, the controlled flow. **In** the case that stopping S4 includes also stopping flow via the draw inlet port *Lᵢₙ,* the same indications apply but without any inlet flow at the draw side 2b. Thus, the outflow from the draw side 2b indicates the controlled flow to the feed inlet port *Eᵢₙ.* Hence, in some embodiments the property is a flow rate of an outflow from the draw side 2b. The method then comprises measuring the outflow from the draw side 2b with a flow sensor (not shown). Thus, the method may include monitoring S6 the property indicative of the controlled flow.

The water permeability of the FO-membrane may thereafter be determined based on one or more of the properties. Hence, the method further comprises determining S7 a water permeability status of the FO-membrane based on the property indicative of the controlled flow. The water permeability status may be determined based on how well the property meets water permeability criteria for a FO-membrane with an acceptable water permeability status. Hence, in some embodiments, determining S7 comprises determining whether the property meets one or more criteria for a FO-membrane with an acceptable water permeability status. For example, the method may comprise evaluating characteristics of the property, such as gradient or magnitude. For a FO-membrane with an acceptable water permeability status, an expected flow rate of inflow to or outflow from the feed inlet port *Eᵢₙ* may be determined, which maintains the TMP constant at the predetermined TMP, and at the same operating conditions. An acceptable flow rate for a FO-membrane with acceptable water permeability status may then be established as being inside or at an interval around this determined flow rate. A controlled flow rate inside or at the interval is then an indication that the FO-membrane has an acceptable permeability status. A controlled flow rate outside the interval that is too high is an indication of a leak, while a flow rate outside the interval that is too low flow indicates poor water permeability. The same applies for the flow out from the draw side 2b. Hence, in some embodiments, the method comprises determining that the FO-membrane has an acceptable water permeability status upon determining that the property is within or at a predetermined interval defining a FO-membrane having an acceptable water permeability status, or else determining that the FO-membrane has a water permeability error. Hence, if the property is within or at an acceptable interval for the property, the method comprises determining that the FO-membrane has an acceptable water permeability status and thus no water permeability error. If the property is outside the acceptable interval, the method comprises determining that the FO-membrane has a water permeability error. The property may alternatively be compared with the expected predetermined value of the same property determined with a FO-membrane with an acceptable water permeability status under the same operating conditions. A result of the comparison reveals if the FO-membrane has an acceptable water permeability status or not. Having the same operating conditions includes the same hydrostatic pressures and the same predetermined TMP to control towards. It may also include the same flow rates. The expected predetermined value may be experimentally determined or be determined based on calculations and/or assumptions.

Next, a second embodiment of testing of water permeability by stopping and controlling flow to/from the feed side 2a is explained. The first and second embodiment are for the most part the same, except for the features explained in the following. To perform the testing of water permeability by stopping and controlling flow to/from the feed side 2a according to second embodiment, the method comprises stopping S4 flow via the feed inlet port *Eᵢₙ.* The stopping of the flow via the feed inlet port *Eᵢₙ* is illustrated in Fig. 4B with a filled inlet port *Eᵢₙ.* **In** the examples in Figs. 2 and 3, the flow via the feed inlet port *Eᵢₙ* can be stopped by stopping feed pump 3 and closing the third effluent inlet valve 10c and fourth effluent inlet valve 10d. Flow is allowed through the other ports, as illustrated in Fig. 4B by being not filled. Hence, the flows via the draw outlet port *Lₒᵤₜ* and the draw inlet port *Lᵢₙ* remain unstopped. **In** an alternative embodiment, the flow through the draw inlet port *Lᵢₙ* is also stopped. The method further comprises controlling S5 flow via, either inflow to or outflow from, the feed outlet port *Eₒᵤₜ* based on the monitored one or more pressures, such that the TMP is maintained constant and at a non-zero magnitude. Controlling S5 is for example performed using a drain pump (not shown), arranged to the effluent outlet line 20d. The drain pump may, during production of dialysis fluid, be used for maintaining a certain hydrostatic pressure at the feed side 2a but may alternatively be used for pumping fluid to the feed side 2a. Fluid is then pumped from a drain container (not shown) connected to the effluent outlet line 20d. Stopping S4 flow via the feed inlet port *Eᵢₙ* and controlling S5 inflow to or outflow from the feed outlet port *Eₒᵤₜ* are performed simultaneously. After a short time period of stabilization, the inflow to or outflow from the feed outlet port *Eₒᵤₜ* reveals if there is a water permeability error. The flow may be determined based on a property indicative of the controlled flow. **In** some embodiments, the property is a speed of the drain pump or power provided to the drain pump. **In** some embodiments, the property is a flow rate of the controlled inflow or outflow provided by the drain pump. The remaining features are the same as described for the first embodiment of the method for testing a water permeability by stopping and controlling flow to/from the feed side 2a.

Second, testing of water permeability by stopping and controlling flow to/from the draw side 2b is now explained. All description for the testing of water permeability by stopping and controlling flow to/from the feed side 2a is applicable also to testing of water permeability by stopping and controlling flow to the draw side 2b, except for the changes indicated below. To perform the testing of water permeability by stopping and controlling flow to/from the draw side 2b, in a third embodiment, the method comprises stopping S4 flow via the draw outlet port *Lₒᵤₜ.* The stopping of the flow via the draw outlet port *Lₒᵤₜ* is illustrated in Fig. 4C with a filled outlet port *Lₒᵤₜ.* **In** the examples in Figs. 2 and 3, the flow via the draw outlet port *Lₒᵤₜ* can be stopped by closing the first connecting valve 10h. Flow is allowed through the other ports, as illustrated in Fig. 4C by being not filled. Hence, the flows via the feed outlet port *Eₒᵤₜ* and the feed inlet port *Eᵢₙ* remain unstopped, and typically remain at the same flow rate and pressure as during the step S1. **In** an alternative embodiment, the flow through the feed inlet port *Eᵢₙ* is also stopped, e.g., by stopping pumping with feed pump 3 and closing the third effluent inlet valve 10c and the fourth effluent inlet valve 10d. The method further comprises controlling S5 flow via, either inflow to or outflow from, the draw inlet port *Lᵢₙ* based on the monitored one or more pressures, such that the TMP is maintained constant and at a non-zero magnitude. Controlling S5 is for example performed using the draw pump 5. To maintain the TMP constant means controlling the TMP such that a predetermined TMP value is achieved, by means of controlling the inflow or outflow to the draw side 2b. The only way pure water at the draw side 2b can leave the draw side 2b to the feed side 2a is through a leakage in the FO-membrane 2c and/or via water permeation through the intended water channels in the FO-membrane 2c. To allow any water transport from draw side 2b to feed side 2a through a leakage, the TMP has to be negative, thus, the hydrostatic pressure P_{draw} is greater than the hydrostatic pressure P_{feed}, as TMP = P_{feed} - P_{draw}. The predetermined TMP is then for example from -0.5 to -2 bar (-7.3 to -29 psig), for example, -1 bar (-14.5 psig). P_{feed} typically remains at around atmospheric pressure. For example, the feed side 2a may be open to drain (hence atmospheric pressure) or P_{feed} may be controlled to atmospheric pressure or any other desired pressure. If fluid is transported from the draw side 2b to the feed side 2a and no inflow is allowed to the draw side 2b, P_{draw} will decrease and consequently the TMP will increase. Hence, in some embodiments, the method includes controlling S5 inflow to the draw inlet port *Lᵢₙ* based on the monitored one or more pressures, such that the TMP is maintained at a negative predetermined TMP. Alternatively, the TMP can be positive, which means that P_{feed} is greater than P_{draw} and the TMP is driving the water transport from the feed side 2a to the draw side 2b. The predetermined TMP is then for example from 2 to 5 bar (29 to 72.5 psig), for example, 4 bar (58 psig). Water at the feed side 2a can now leave the feed side 2a via the FO-membrane 2c to the draw side 2a, via a leakage and/or via water permeation through the intended water channels in the FO-membrane 2c. However, the pressure at the draw side 2b increases and water has to be pumped out from the draw side 2b to maintain the TMP constant. Hence, in some embodiments, the method includes controlling S5 outflow from the draw inlet port *Lᵢₙ* based on the monitored one or more pressures, such that the TMP is maintained at a positive predetermined TMP. By controlling the inflow or outflow to the draw side 2b to maintain the TMP constant, the flow to or from the draw side 2b reflects an eventual flow through a leakage and/or membrane water permeation of the FO-membrane 2c.

Stopping S4 flow via the draw outlet port *Lₒᵤₜ* and controlling S5 inflow to or outflow from the draw inlet port *Lᵢₙ* are performed simultaneously in one embodiment. After a short time period of stabilization, the inflow to or outflow from the draw inlet port *Lᵢₙ* reveals if there is a leakage and/or altered membrane permeability. The inflow or outflow may be determined based on a property indicative of the controlled flow. **In** some embodiments, the property is a speed of the draw pump 5 or power provided to the draw pump 5. **In** some embodiments, the property is a controlled flow rate provided by the draw pump 5. Such properties may be readily available as control parameters or other parameters stored in the control arrangement 50 to be used for an evaluation. The flow rate of the controlled flow may alternatively be measured with a flow sensor (not shown). A water transport from the draw side 2b to the feed side 2a is detected as an increased flow through the feed outlet port *Eₒᵤₜ* compared to the flow through the feed inlet port *Eᵢₙ* (provided with the feed pump 3). Thus, the outflow from the feed side 2a indicates the controlled flow to the draw inlet port *Liₙ.* **In** more detail, the difference between the flow rate of the fluid from the feed outlet port *Eₒᵤₜ* and the flow rate of the fluid to the feed inlet port *Eᵢₙ* indicates, e.g., be equal to, the controlled flow. **In** the case that stopping S4 includes also stopping flow via the feed inlet port *Eᵢₙ,* the same applies but without any inlet flow at the feed side 2a. Thus, the outflow from the feed side 2a indicates the controlled flow to the draw inlet port *Lᵢₙ.* Hence, in some embodiments the property is a flow rate of an outflow from the feed side 2a. The method then comprises measuring the outflow from the feed side 2a with a flow sensor (not shown). Thus, the method may include monitoring S6 the property indicative of the controlled flow.

The monitoring S6 and the determining S7 may be performed as previously explained, with the following change: For a FO-membrane having an acceptable water permeability status, an expected flow rate of inflow to or outflow from the draw inlet port *Lᵢₙ* may be determined that will keep the TMP constant at the predetermined TMP, at the same operating conditions. An acceptable flow rate for a FO-membrane with acceptable water permeability status may then be established as being inside or on an interval around this determined flow rate. A controlled flow rate inside or on the interval is then an indication that the FO-membrane has an acceptable water permeability status. A controlled flow rate outside the interval that is too high is an indication of a leak, while a flow rate outside the interval that is too low flow indicates poor water permeability. The same applies for the flow out from the feed side 2a.

Next, a fourth embodiment for testing a water permeability by stopping and controlling flow to/from the draw side 2b is explained. The third and fourth embodiments are for the most part the same, except for the features explained in the following. To perform the testing of water permeability by stopping and controlling flow to/from the draw side 2b in the fourth embodiment, the method comprises stopping S4 flow via the draw inlet port *Lᵢₙ.* The stopping of the flow via the draw inlet port *Lᵢₙ* is illustrated in Fig. 4D with a filled inlet port *Lᵢₙ.* **In** the examples in Figs. 2 and 3, the flow via the draw inlet port *Lᵢₙ* can be stopped by stopping the draw pump 5. Flow is allowed through the other ports, as illustrated in Fig. 4D by being not filled. Hence, the flows via the feed outlet port *Eₒᵤₜ* and the feed inlet port *Eᵢₙ* remain unstopped. **In** an alternative embodiment, the flow through the feed inlet port *Eᵢₙ* is also stopped. The method further comprises controlling S5 flow via, either inflow to or outflow from, the draw outlet port *Lₒᵤₜ* based on the monitored one or more pressures, such that the TMP is maintained constant and at a non-zero magnitude. Controlling S5 is for example performed using the diluted concentrate pump 6 and another valve (not shown) connected to the diluted dialysis concentrate line 20g arranged between the diluted dialysis concentrate container 32 and a connection point of the first connecting line 20h to the diluted dialysis concentrate line 20g, which is closed.

Stopping S4 flow via the draw inlet port *Lᵢₙ* and controlling S5 flow via the draw outlet port *Lₒᵤₜ* are performed simultaneously in one embodiment. After a short time period of stabilization, the inflow to or outflow from the draw outlet port *Lₒᵤₜ* reveals if there is a water permeability error from the feed side 2a to the draw side 2b. The flow may be determined based on a property indicative of the controlled flow. **In** some embodiments, the property is a speed of the diluted concentrate pump 6 or power provided to the diluted concentrate pump 6. **In** some embodiments, the property is a flow rate of the controlled flow provided by the diluted concentrate pump 6. Such properties may be readily available as control parameters or other parameters in the control arrangement 50 to be used for a determination. The flow rate of the controlled flow may alternatively be measured with a flow sensor (not shown). The remaining features are the same as for the third embodiment of the method for testing of water permeability by stopping and controlling flow to/from the draw side 2b.

According to some embodiments, the method comprises determining a water permeability status by testing water permeability from feed side 2a to draw side 2b and by testing water permeability from draw side 2b to feed side 2a. Such a method may include performing the method for both a positive TMP and a negative TMP. Thereby, a more thorough test of leakage is performed. Hence, in some embodiments, the method comprising performing the method for both controlling flow S5 such that the TMP is maintained positive and such that TMP is maintained negative, and wherein the determining S7 further comprises determining a water permeability status of the FO-membrane based on a property indicative of the controlled flow for each case. The method may be performed using any of the explained embodiments herein having a positive TMP and a negative TMP, respectively.

The result of the determination may be communicated to a user via a user interface (not shown) of the control arrangement 10, and/or an alarm may be initiated if a water permeability error is detected. The user may then take appropriate action, such as replacing the FO-device to correct the water permeability error.

The disclosure also relates to a control arrangement 10 for determining a water permeability status of a forward osmosis FO membrane 2c of a FO device 2 in a dialysis fluid generation apparatus 1. The control arrangement 10 comprises a feed pump 3 configured to provide a flow of pure water at the feed side 2a. The control arrangement 10 comprises a draw pump 5 configured to provide a flow of pure water at the draw side 2b. The control arrangement 10 further comprises one or more valves 10 configured to control flow via one or more of the ports, and one or more pressure sensors 8a, 8b configured to sense one or more pressures indicative of a transmembrane pressure (TMP) between the feed side 2a and the draw side 2b. The control arrangement 10 is further configured to monitor one or more pressures indicative of the transmembrane pressure (TMP). The control arrangement 10 is also configured to stop flow via one of the ports and to control flow to or from the one of the feed side 2a and the draw side 2b where flow has been stopped, based on the one or more pressures, such that the TMP is maintained constant and at a non-zero magnitude. The control arrangement 10 is further configured to determine water permeability status of the FO-membrane based on a property indicative of the controlled flow. According to some embodiments, the control arrangement 10 is configured to perform the method according to any one of the embodiments described herein, alone or in combination with other embodiments or parts thereof.

While the invention has been described in connection with what are presently considered to be the most practical and preferred embodiments, it is to be understood that the invention is not to be limited to the disclosed embodiments, but on the contrary, is intended to cover various modifications and equivalent arrangements included within the scope of the appended claims.

## Claims

1. A method for determining water permeability of a forward osmosis, FO, membrane (2c) of a FO device (2) in a dialysis fluid generation apparatus (1), the FO-membrane (2c) separating a feed side (2a) and a draw side (2b) of the FO device (2), the FO-device (2) comprising a feed inlet port *(Eᵢₙ)* and a feed outlet port *(Eₒᵤₜ)* in fluid communication with the feed side (2a), and a draw inlet port *(Lᵢₙ)* and a draw outlet port *(Lₒᵤₜ)* in fluid communication with the draw side (2b), the method comprising:
providing (S1) a flow of pure water at the feed side (2a);
providing (S2) a flow of pure water at the draw side (2b);
monitoring (S3) one or more pressures indicative of a transmembrane pressure, TMP, between the feed side (2a) and the draw side (2b);
stopping (S4) flow via one of the ports;
controlling (S5) flow, with a pump (3, 5), to or from the one of the feed side (2a) and the draw side (2b) that has a stopped flow based on the monitored one or more pressures, such that the TMP is maintained constant and at a non-zero magnitude; and
determining (S7) the water permeability of the FO-membrane based on a property indicative of the controlled flow, wherein the property is any one of a speed of the pump (3, 5), power provided to the pump (3, 5), a flow rate of the controlled flow to the one of the feed side (2a) or the draw side (2b) that has a stopped flow, or a flow rate of an outflow from the other one of the feed side (2a) and the draw side (2b).

2. The method according to claim 1, comprising monitoring (S6) the property indicative of the controlled flow.

3. The method according to any one of the preceding claims, wherein determining the water permeability (S7) of the FO-membrane comprises determining whether the property meets one or more criteria for a FO-membrane having an acceptable water permeability.

4. The method according to claim 3, comprising determining that the FO-membrane has an acceptable water permeability upon determining that the property is within or at a predetermined interval defining a FO-membrane having an acceptable water permeability, or else determining that the FO-membrane has a water permeability error.

5. The method according to any one of the preceding claims, comprising performing the method for both controlling (S5) flow such that the TMP is maintained positive and such that TMP is maintained negative, and wherein the determining (S7) further comprises determining the water permeability of the FO-membrane based on a property indicative of the controlled flow for each case.

6. The method according to any one of the preceding claims, wherein the controlling (S5) flow comprises controlling flow such that TMP is maintained at a predetermined constant TMP.

7. A control arrangement (10) for determining a water permeability of a forward osmosis, FO, membrane (2c) of a FO device (2) in a dialysis fluid generation apparatus (1), the FO-membrane (2c) separating a feed side (2a) and a draw side (2b) of the FO device (2), the FO-device (2) comprising a feed inlet port *(Eᵢₙ)* and a feed outlet port *(Eₒᵤₜ)* in fluid communication with the feed side (2a), and a draw inlet port *(Lᵢₙ)* and a draw outlet port *(Lₒᵤₜ)* in fluid communication with the draw side (2b), the control arrangement (10) comprising:
a feed pump (3) configured to provide a flow of pure water at the feed side (2a);
a draw pump (5) configured to provide a flow of pure water at the draw side (2b);
one or more valves (10) configured to control flow via one or more of the ports;
one or more pressure sensors (8a, 8b) configured to sense one or more pressures indicative of a transmembrane pressure, TMP, between the feed side (2a) and the draw side (2b);
wherein the control arrangement (10) is configured to:
stop flow via one of the ports;
monitor one or more pressures indicative of the TMP;
control flow, with a pump (3, 5), to or from the one of the feed side (2a) and the draw side (2b) that has a stopped flow, based on the one or more pressures, such that the TMP is maintained constant and at a non-zero magnitude; and
determine the water permeability of the FO-membrane based on a property indicative of the controlled flow, wherein the property is any one of a speed of the pump (3, 5), power provided to the pump (3, 5), a flow rate of the controlled flow to the one of the feed side (2a) or the draw side (2b) that has a stopped flow, or a flow rate of an outflow from the other one of the feed side (2a) and the draw side (2b).

8. The control arrangement (10) according to claim 7, which is configured to perform the method according to any one of the claims 2 to 6.

9. A solution generation apparatus (1) for generating dialysis solution, the apparatus (1) comprising a forward osmosis device (2) comprising a FO-membrane (2c) that separates a feed side (2a) and a draw side (2b) of the FO device (2), the apparatus further comprising a control arrangement (10) according to claim 7 or 8.

## Patentansprüche

1. Verfahren zum Bestimmen von Wasserdurchlässigkeit einer Vorwärtsosmose(FO)-Membran (2c) einer FO-Vorrichtung (2) in einer Vorrichtung (1) zur Erzeugung eines Dialysefluids, wobei die FO-Membran (2c) eine Zufuhrseite (2a) und eine Zugseite (2b) der FO-Vorrichtung (2) voneinander trennt, wobei die FO-Vorrichtung (2) eine Zufuhreinlassöffnung (Eᵢₙ) und eine Zufuhrauslassöffnung *(Eₒᵤₜ)* in Fluidverbindung mit der Zufuhrseite (2a) und eine Zugeinlassöffnung *(Lᵢₙ)* und eine Zugauslassöffnung (Lₒᵤₜ) in Fluidverbindung mit der Zugseite (2b) umfasst, das Verfahren umfassend:
Bereitstellen (S1) eines Stroms von reinem Wasser an der Zufuhrseite (2a);
Bereitstellen (S2) eines Stroms von reinem Wasser an der Zugseite (2b);
Überwachen (S3) von einem oder mehreren Drücken, die auf einen Transmembrandruck (TMP) zwischen der Zufuhrseite (2a) und der Zugseite (2b) hinweisen;
Stoppen (S4) des Stroms über eine der Öffnungen;
Steuern (S5) des Stroms mit einer Pumpe (3, 5) zu oder von der einen aus der Zufuhrseite (2a) und der Zugseite (2b), die basierend auf dem überwachten einen oder den überwachten mehreren Drücken einen gestoppten Strom aufweist, sodass der TMP konstant gehalten wird und auf einer Größe ungleich Null gehalten wird; und
Bestimmen (S7) der Wasserdurchlässigkeit der FO-Membran basierend auf einer Eigenschaft, die den gesteuerten Strom angibt, wobei die Eigenschaft eine ist aus einer Geschwindigkeit der Pumpe (3, 5), einer Leistung, die an die Pumpe (3, 5) geliefert wird, einer Flussrate des gesteuerten Stroms zu der einen aus der Zufuhrseite (2a) und der Zugseite (2b), die einen gestoppten Strom aufweist, oder einer Flussrate eines Abflusses von der anderen der Zufuhrseite (2a) und der Zugseite (2b).

2. Verfahren nach Anspruch 1, umfassend Überwachen (S6) der Eigenschaft, die den gesteuerten Strom angibt.

3. Verfahren nach einem der vorhergehenden Ansprüche, wobei das Bestimmen der Wasserdurchlässigkeit (S7) der FO-Membran das Bestimmen umfasst, ob die Eigenschaft ein oder mehrere Kriterien für eine FO-Membran mit einer akzeptablen Wasserdurchlässigkeit erfüllt.

4. Verfahren nach Anspruch 3, umfassend Bestimmen, dass die FO-Membran eine akzeptable Wasserdurchlässigkeit aufweist, wenn bestimmt wird, dass die Eigenschaft eine FO-Membran mit einer akzeptablen Wasserdurchlässigkeit definiert oder in einem vorbestimmten Intervall davon liegt, oder aber Bestimmen, dass die FO-Membran einen Wasserdurchlässigkeitsdefekt aufweist.

5. Verfahren nach einem der vorhergehenden Ansprüche, umfassend das Durchführen des Verfahrens sowohl zum Steuern (S5) des Stroms so, dass der TMP positiv gehalten wird, als auch so, dass der TMP negativ gehalten wird, und wobei das Bestimmen (S7) ferner das Bestimmen der Wasserdurchlässigkeit der FO-Membran basierend auf einer Eigenschaft umfasst, die den gesteuerten Strom für den jeweiligen Fall angibt.

6. Verfahren nach einem der vorhergehenden Ansprüche, wobei das Steuern (S5) des Stroms ein Steuern des Stroms derart umfasst, dass der TMP auf einem vorbestimmten konstanten TMP gehalten wird.

7. Steuerungsanordnung (10) zum Bestimmen einer Wasserdurchlässigkeit einer Vorwärtsosmose(FO)-Membran (2c) einer FO-Vorrichtung (2) in einer Vorrichtung (1) zur Erzeugung eines Dialysefluids, wobei die FO-Membran (2c) eine Zufuhrseite (2a) und eine Zugseite (2b) der FO-Vorrichtung (2) voneinander trennt, wobei die FO-Vorrichtung (2) eine Zufuhreinlassöffnung (Eᵢₙ) und eine Zufuhrauslassöffnung *(Eₒᵤₜ)* in Fluidverbindung mit der Zufuhrseite (2a) und eine Zugeinlassöffnung *(Lᵢₙ)* und eine Zugauslassöffnung (Lₒᵤₜ) in Fluidverbindung mit der Zugseite (2b) umfasst, die Steuerungsanordnung (10) umfassend:
eine Zufuhrpumpe (3), dazu ausgelegt, einen Strom von reinem Wasser an der Zufuhrseite (2a) bereitzustellen;
eine Zugpumpe (5), dazu ausgelegt, einen Strom von reinem Wasser an der Zugseite (2b) bereitzustellen;
ein oder mehrere Ventile (10), dazu ausgelegt, den Strom über eine oder mehrere der Öffnungen zu steuern;
einen oder mehrere Drucksensoren (8a, 8b), ausgelegt zum Erfassen von einem oder mehreren Drücken, die auf einen Transmembrandruck (TMP) zwischen der Zufuhrseite (2a) und der Zugseite (2b) hinweisen;
wobei die Steuerungsanordnung (10) ausgelegt ist zum:
Stoppen des Stroms über eine der Öffnungen;
Überwachen eines oder mehrerer Drücke, die auf den TMP hinweisen;
Steuern des Stroms mit einer Pumpe (3, 5) zu oder von der einen aus der Zufuhrseite (2a) und der Zugseite (2b), die basierend auf dem einen oder den mehreren Drücken einen gestoppten Strom aufweist, sodass der TMP konstant gehalten wird und auf einer Größe ungleich Null gehalten wird; und
Bestimmen der Wasserdurchlässigkeit der FO-Membran basierend auf einer Eigenschaft, die den gesteuerten Strom angibt, wobei die Eigenschaft eine ist aus einer Geschwindigkeit der Pumpe (3, 5), einer Leistung, die an die Pumpe (3, 5) geliefert wird, einer Flussrate des gesteuerten Stroms zu der einen aus der Zufuhrseite (2a) und der Zugseite (2b), die einen gestoppten Strom aufweist, oder einer Flussrate eines Abflusses von der anderen der Zufuhrseite (2a) und der Zugseite (2b).

8. Steuerungsanordnung (10) nach Anspruch 7, ausgelegt zum Durchführen des Verfahrens nach einem der Ansprüche 2 bis 6.

9. Lösungserzeugungsvorrichtung (1) zum Erzeugen einer Dialyselösung, wobei die Vorrichtung (1) eine Vorwärtsosmosevorrichtung (2) mit einer FO-Membran (2c), die eine Zufuhrseite (2a) und eine Zugseite (2b) der FO-Vorrichtung (2) voneinander trennt, umfasst, wobei die Vorrichtung ferner eine Steuerungsanordnung (10) nach Anspruch 7 oder 8 umfasst.

## Revendications

1. Procédé destiné à déterminer la perméabilité à l'eau d'une membrane d'osmose directe, FO (2c) d'un dispositif FO (2) dans un appareil de production de fluide de dialyse (1), la membrane FO (2c) séparant un côté alimentation (2a) et un côté extraction (2b) du dispositif FO (2), le dispositif FO (2) comprenant un orifice d'entrée d'alimentation (Eᵢₙ) et un orifice de sortie d'alimentation *(Eₒᵤₜ)* en communication fluidique avec le côté alimentation (2a), et un orifice d'entrée d'extraction *(Lᵢₙ)* et un orifice de sortie d'extraction (Lₒᵤₜ) en communication fluidique avec le côté extraction (2b), le procédé comprenant :
la fourniture (S1) d'un écoulement d'eau pure côté alimentation (2a) ;
la fourniture (S2) d'un écoulement d'eau pure côté extraction (2b) ;
la surveillance (S3) d'une ou plusieurs pressions indicatives d'une pression transmembranaire, TMP, entre le côté alimentation (2a) et le côté extraction (2b) ;
l'arrêt (S4) de l'écoulement par un des orifices ;
la régulation (S5), avec une pompe (3, 5), de l'écoulement vers ou depuis celui du côté alimentation (2a) ou du côté extraction (2b) dont l'écoulement est arrêté en fonction de la ou des pressions surveillées de telle sorte que la TMP soit maintenue constante et à une amplitude non nulle ; et
la détermination (S7) de la perméabilité à l'eau de la membrane FO en fonction d'une propriété indicative de l'écoulement régulé, la propriété étant n'importe laquelle parmi une vitesse de la pompe (3, 5), une puissance fournie à la pompe (3, 5), un débit de l'écoulement régulé vers celui du côté alimentation (2a) ou du côté extraction (2b) dont l'écoulement est arrêté, et un débit d'un écoulement sortant depuis l'autre côté parmi le côté alimentation (2a) et le côté extraction (2b).

2. Procédé selon la revendication 1, comprenant la surveillance (S6) de la propriété indicative de l'écoulement régulé.

3. Procédé selon l'une quelconque des revendications précédentes, dans lequel la détermination de la perméabilité à l'eau (S7) de la membrane FO comprend la détermination que la propriété respecte ou non un ou plusieurs critères pour une membrane FO ayant une perméabilité à l'eau acceptable.

4. Procédé selon la revendication 3, comprenant la détermination que la membrane FO a une perméabilité à l'eau acceptable lors de la détermination que la propriété se situe à l'intérieur ou à un intervalle prédéterminé définissant une membrane FO ayant une perméabilité à l'eau acceptable, sinon la détermination que la membrane FO présente une erreur de perméabilité à l'eau.

5. Procédé selon l'une quelconque des revendications précédentes, comprenant la réalisation du procédé destiné à réguler (S5) l'écoulement à la fois de telle sorte que la TMP soit maintenue positive et de telle sorte que la TMP soit maintenue négative, et dans lequel la détermination (S7) comprend en outre la détermination de la perméabilité à l'eau de la membrane FO en fonction d'une propriété indicative de l'écoulement régulé pour chaque cas.

6. Procédé selon l'une quelconque des revendications précédentes, dans lequel la régulation (S5) de l'écoulement comprend la régulation de l'écoulement de telle sorte que la TMP soit maintenue à une TMP constante prédéterminée.

7. Agencement de régulation (10) destiné à déterminer la perméabilité à l'eau d'une membrane d'osmose directe, FO (2c) d'un dispositif FO (2) dans un appareil de production de fluide de dialyse (1), la membrane FO (2c) séparant un côté alimentation (2a) et un côté extraction (2b) du dispositif FO (2), le dispositif FO (2) comprenant un orifice d'entrée d'alimentation (Eᵢₙ) et un orifice de sortie d'alimentation *(Eₒᵤₜ)* en communication fluidique avec le côté alimentation (2a), et un orifice d'entrée d'extraction *(Lᵢₙ)* et un orifice de sortie d'extraction (Lₒᵤₜ) en communication fluidique avec le côté extraction (2b), l'agencement de commande (10) comprenant :
une pompe d'alimentation (3) conçue pour fournir un écoulement d'eau pure côté alimentation (2a) ;
une pompe d'extraction (5) conçue pour fournir un écoulement d'eau pure côté extraction (2b) ;
une ou plusieurs vannes (10) conçues pour réguler l'écoulement par un ou plusieurs des orifices ;
un ou plusieurs capteurs de pression (8a, 8b) conçus pour détecter une ou plusieurs pressions indicatives d'une pression transmembranaire, TMP, entre le côté alimentation (2a) et le côté extraction (2b) ;
l'agencement de régulation (10) étant conçu pour :
arrêter l'écoulement par un des orifices ;
surveiller une ou plusieurs pressions indicatives de la TMP ;
réguler, avec une pompe (3, 5), l'écoulement vers ou depuis celui du côté alimentation (2a) ou du côté extraction (2b) dont l'écoulement est arrêté en fonction de la ou des pressions de telle sorte que la TMP soit maintenue constante et à une amplitude non nulle ; et
déterminer la perméabilité à l'eau de \la membrane FO en fonction d'une propriété indicative de l'écoulement régulé, la propriété étant n'importe laquelle parmi une vitesse de la pompe (3, 5), une puissance fournie à la pompe (3, 5), un débit de l'écoulement régulé vers celui du côté alimentation (2a) ou du côté extraction (2b) dont l'écoulement est arrêté, et un débit d'un écoulement sortant depuis l'autre côté parmi le côté alimentation (2a) et le côté extraction (2b).

8. Agencement de régulation (10) selon la revendication 7, qui est conçu pour réaliser le procédé selon l'une quelconque des revendications 2 à 6.

9. Appareil de production de solution (1) destiné à produire une solution de dialyse, l'appareil (1) comprenant un dispositif d'osmose directe (2) comprenant une membrane FO (2c) qui sépare un côté alimentation (2a) et un côté extraction (2b) du dispositif FO (2), l'appareil comprenant en outre un agencement de régulation (10) selon la revendication 7 ou 8.
